# EUROPEAN PATENT APPLICATION

(11) **EP 1 495 717 A1**
(43) Date of publication of application: **12.01.2005**
(21) Application number: 02805492.2
(22) Date of filing: 23.12.2002
(51) Int. Cl.: A61B 5/05

(54) **METHOD FOR DIAGNOSIS OF HUMAN ORGANISM**

(30) Priority: 21.12.2001 RU 2001135967
(71) Applicant: Korotkov, Konstantin Georgievich, St.Petersburg, 191040 (RU)
(72) Inventor: The designation of the inventor has not yet been filed
(74) Representative: Smaggasgale, Gillian Helen
(86) International application number: PCT/RU2002/000569
(87) International publication number: WO 2003/053240

(57) **Abstract**

The invention relates to medicine and can be used for the disclosure of diseases in early stages.

An object of the present invention is simplification and increase of accuracy of diagnosis of the state of human organism.

According to the invention in the method of diagnosis of the human organism state consisting in obtaining visual images of structure of gas discharge streamers of fingers in electromagnetic field when contacted with electrode and in estimating streamer parameters, the visual images of structure of gas discharge streamers of fingers are divided into sectors, corresponding to various organs or systems of human organism, each sector is projected along the contour of the silhouette of human body, forming a single image at that, after which the human organism state is diagnosed by way of estimating parameters of gas discharge streamers of the obtained single image; closed curves are determined at that, corresponding to the borders of zones of contact of fingers with the electrode, cross points of these lines with the lines dividing the images of structure of gas discharge streamers of fingers into sectors are determined, and the single image is formed by these points along the silhouette of human body.

## Description

### Technical field

This invention relates to medicine and can be applied for the reveal of diseases at early stages.

### Background of the invention

Patent SU, A, 935076 protects a method of diagnosis of vegetovascular crises, according to which the structure of glow of fingertips in high frequency electrical field is repeatedly determined and the probability of crisis is found in compliance with the increase of streamer length by 15-30 % as compared to the initial level.

This method is only applied to reveal the pathologies of patients with vegetovascular dysfunction.

Patent SU, A, 1812965 protects a method of diagnosis of osteochondrosis of the cervical part of spine performed by way of radiation treatment of parts of the human body followed by image registration. The fingertips are radiated by impulse electric field with intensity 10⁶ - 10⁷ V/cm with the registration of Kirlian image; if there are not more than four T-form streamers for one finger and if these are for not more than three fingers of one hand, the diagnosis of osteochondrosis of the cervical part of spine is performed.

The disadvantage of this method is also its narrow orientation - diagnosis of diseases of the cervical part of spine.

Patent RU, A1, 94012892 protects a method of diagnosis of state of the human organism, consisting in the registration of structure of gas discharge streamers of fingers in the electromagnetic field, visualization and estimation of the streamer size. The registration is performed on a layer of thermoplastic material on a dielectric plate, which is then heated up to the temperature of softening of plastic. A phase-contrast visualization of streamers is performed at that, and the size and quantity of streamers are determined in the points of classical acupuncture.

In accordance with this technique, taken as a prototype of the present invention, visual images of gas discharge streamers of all fingers are obtained one after another. In addition, the diagnosis of the organism state is based on the known data on the correlation between the particular parts of glow of one or another finger with the state of various organs and systems. These data are widely known and given, particularly, in the book by P.Mandel "Energy Emission Analysis. New Application of Kirlian Photography for Holistic Medicine", Germany, 1986, as well as in others sources, for example in the book by K.Korotkov "Aura and Consciousness: New Stage of Scientific Understanding", p. 209, St. Petersburg, 1998.

The disadvantage of the prototype is the fact that ten separate images are to be registered and estimated at the same time while performing the diagnosis of the organism state; that makes especially difficult the estimation of changes of the organism state in the process of the therapy and considering time. In this case several tens of the obtained images are to be estimated and compared with one another and with the reference images, which practically excludes the possibility of rigorous diagnosis and increases the risk of error.

### Summary of the invention

An object of the present invention is simplification and increase of accuracy of diagnosis of the human organism state.

According to the invention in the method of diagnosis of the human organism state, consisting in obtaining visual images of structure of gas discharge streamers of fingers in electromagnetic field when contacted with the electrode and estimating streamer parameters, the visual images of the structure of gas discharge streamers of fingers are divided into sectors, corresponding to various organs or systems of human organism, each sector is projected on the contour of the human body silhouette, forming a single image at that, after which the diagnosis of the human state is performed by way of estimation of the parameters of gas discharge streamers of the obtained single image; in addition, closed curves corresponding to the borders of zones of contact of fingers with the electrode can be determined, cross points of these lines with the lines dividing images of the structure of gas discharge streamers of fingers into sectors can be found, and the single image is formed along the contour of the silhouette of human body by these points.

The applicant has not found any sources of information containing data on technical solutions, identical to the claimed one. In the opinion of the applicant, that enables to conclude that the invention conforms to the criterion "novelty" (N).

Realization of distinguishing features of the claimed method, owing to the application of a totality of various projections, enables to receive a single spatial image of structure of gas discharge glow around human body, which increases the informativeness of the method and, correspondingly, the accuracy of diagnosis.

The applicant has not found any information on the influence of distinguishing features of the invention on the achievable technical result The mentioned condition enables to conclude that the claimed invention conforms to the criterion "inventive step" (IS).

### Brief description of the drawings

Hereinafter the invention is illustrated by detailed description of its embodiment with references to drawings as follows.
Fig.1 shows an image of structure of gas discharge streamers of fingers;
Fig.2 shows a single image, made up from image fragments of gas discharge streamers of fingers; frontal projection; corresponds to absence of pathologies in the organism;
Fig.3 illustrates the same as fig. 2, right side projection;
Fig.4 illustrates the same as fig. 2, left side projection;
Fig.5 illustrates the same as fig. 2, presence of expressed pathology of the organism;
Fig.6 illustrates the same as fig. 5, in 30 minutes after the acupuncture session;
Fig. 7 shows images of gas discharge streamers of a finger with the denoted points of concurrence of lines, limiting the zones of contact of fingers with electrode, with the lines dividing the image into sectors.

### Preferred embodiment

The proposed method is embodied as follows.

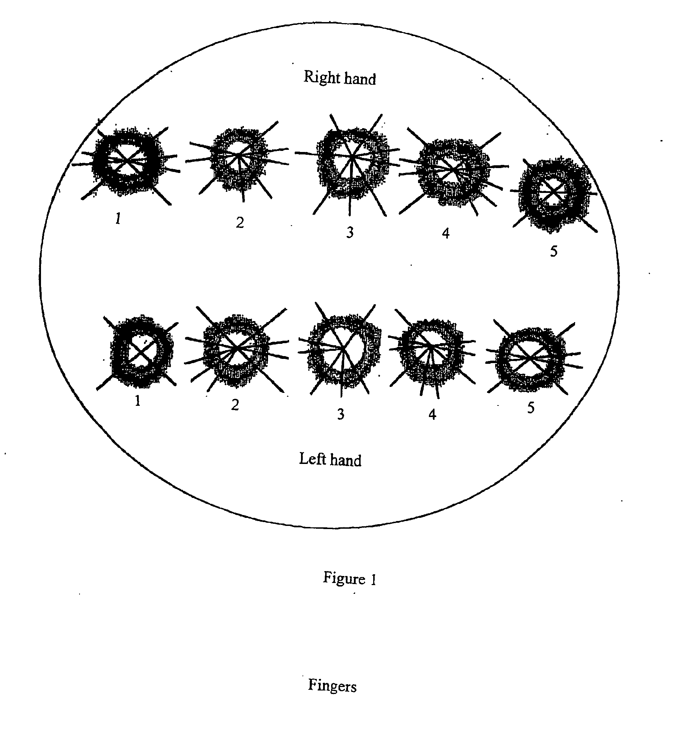

First, visual images of structure of gas discharge streamers of all the ten fingers (fig.1) are obtained (fig.1). That can be performed by any well known method, for example, as follows.

The electromagnetic field with intensity 10⁶-10⁸ V/cm is created on the surface of glass plate by means of electronic pulse generator with amplitude 10-20 kV, duration 10msec, duty ratio 1000 Hz, with pulses fed by pulse strings 0.5 sec long, by electrode performed in the form of a layer of optically transparent material (in the given case - thin layer of SnO₂ 200 mcm thick). In the particular embodiment the electrical pulse generator "Correx" is applied, manufactured by Russian company "Kirlionics Technologies International Ltd." (St. Petersburg). The fingers contact the surface of the glass plate one after another. The electromagnetic field produces gas discharge glow around the finger. This glow is transferred by means of objective lens to optoelectronic digital converter, where it's transformed into a digital code. In this case the converter represents a matrix structure made on the basis of a charged coupled device (the so-called CCD-structure). The signal comes from the output of the optoelectronic digital converter to the computer input, where quantitative parameters of structure of gas discharge glow around each finger are determined. In the particular embodiment parameters reflecting two-dimensional geometrical characteristics of glow structures, as well as brightness characteristics, are determined. Gas discharge glow around finger can be represented in the form of two-dimensional color image in computer display.

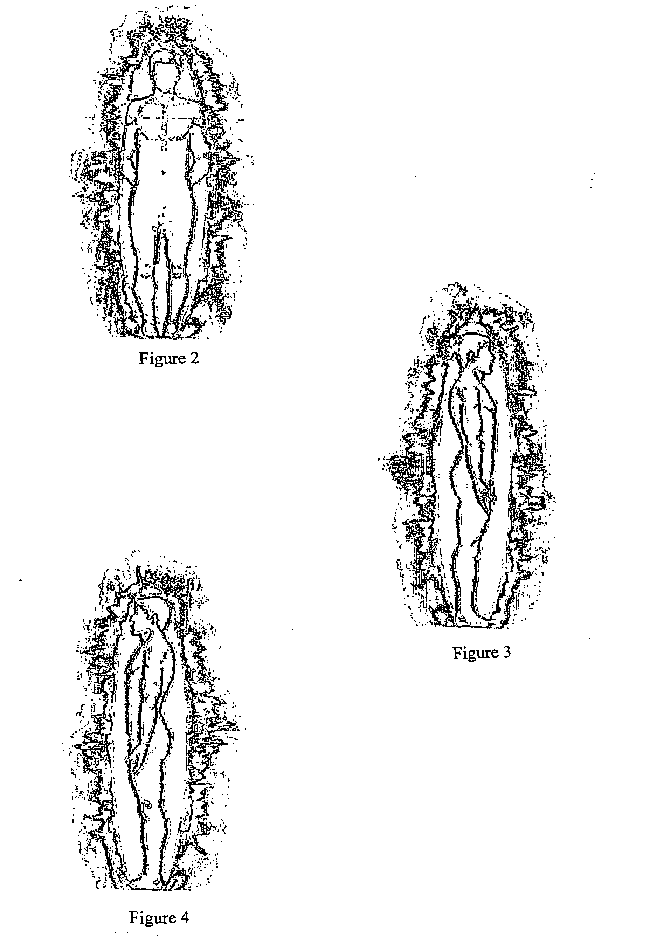

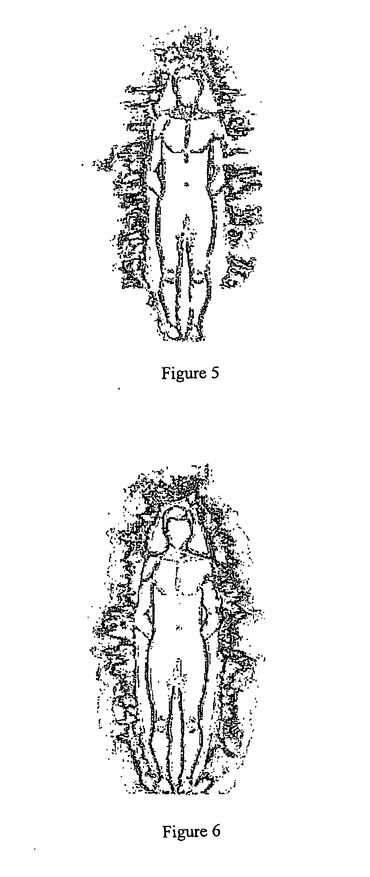

Then the obtained visual images of the structure of gas discharge streamers of each finger are divided into sectors, corresponding to various organs or systems of human organism, by lines 1 and each sector is projected along the contour of the silhouette of human body. That can be done only in frontal projection or in some other additional projections. Single images corresponding to the human body are formed, representing a totality of separate fragments (sectors) of images of structure of gas discharge streamers of fingers. Reference image (corresponding to the absence of expressed pathologies of organism) is given in fig. 2, 3, 4, and the image corresponding to the presence of expressed pathology is given in fig.5.

In the particular embodiment the single image is formed by computer technique, although it can be formed by other means, for example, with the help of usual graphic constructions.

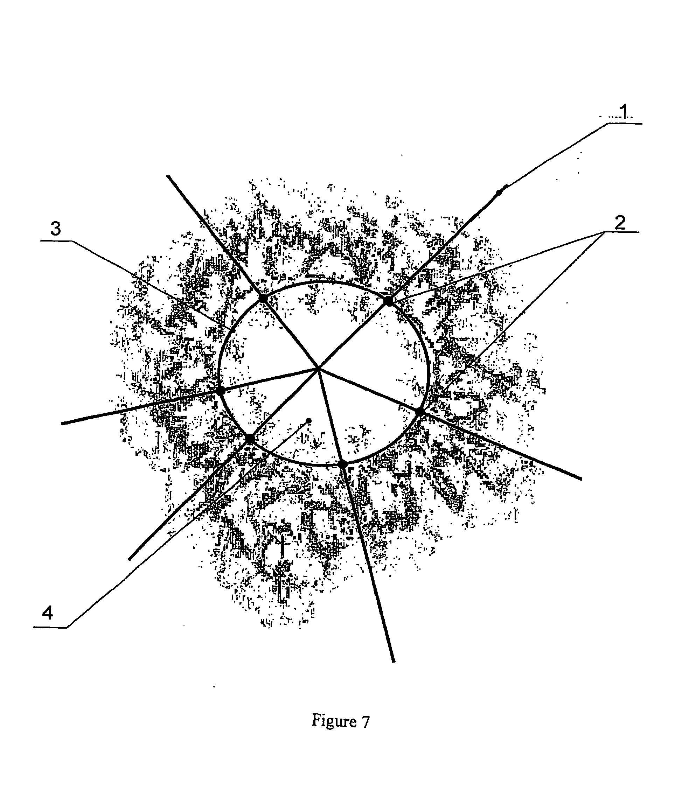

The accuracy of building single image rises when it is formed by the contour of silhouette of the human body by 2 points of concurrence of lines 1, dividing the images of structure of gas discharge streamers of fingers into sectors, with closed curves 3, corresponding to the border of zone 4 of contact of finger with the electrode (fig. 7).

The state of the organism is diagnosed and pathology of particular organs and systems is revealed by way of estimation of parameters of gas discharge streamers of the obtained single image. This estimation is done in comparison with the reference image. There is a possibility to estimate and compare not separate numerous images, corresponding to the fingers, but single complex images, corresponding to the general silhouette of the human body. This significantly simplifies the control of the organism in dynamics, for example, in the process of therapy, and enables to accurately record and estimate the changes which take place.

In the particular embodiment, the image looks according to fig.6 after therapy, particularly, an acupuncture session, directed to the correction of patient's state, shown in fig.5. Direct comparison of both images enables to rapidly and quite graphically estimate the changes which took place as a result of psychotherapy. At the same time, comparing the image in fig.6 with the reference image (fig.1), it can be concluded that the performed therapy in the volume as it was performed did not provide complete (or satisfactory) correction of organism state; certain conclusions with respect to tactics of further treatment can be made. Thus, the proposed method enables to quickly, visually and quite accurately determine the initial state of the organism and follow the changes of this state. The observed changes of state can be estimated both qualitatively and quantitatively by way of calculating geometrical, fractal, brightness, etc. parameters of the compared images.

Application of a totality of various projections enables to obtain a single spatial image of structure of the gas discharge glow around human body, which improves the informativeness of method and, correspondingly, the accuracy of diagnosis.

### Industrial applicability

Known materials and equipment produced in factory conditions were applied for the realization of the method, which stipulates that the invention corresponds to the criterion "industrial applicability" (IA).

## Claims

1. Method of diagnosis of the human organism state, consisting in obtaining visual images of structure of gas discharge streamers of fingers in electromagnetic field, when contacted with electrode, and in estimating streamer parameters, wherein the visual images of structure of gas discharge streamers of fingers are divided into sectors, corresponding to various organs or systems of the human organism, each sector is projected along the contour of the silhouette of the human body, forming the single image at that, after which the human organism state is diagnosed by way of estimation of parameters of gas discharge streamers of the obtained single image.

2. Method OF Claim 1, wherein closed curves are determined, corresponding to the borders of zones of contact of fingers with the electrode, cross points of these lines with the lines, dividing images of structure of gas discharge streamers of fingers into sectors are determined, single image is formed by these points along the contour of the silhouette of the human body, at that.
